# EUROPEAN PATENT SPECIFICATION

(11) **EP 3 402 410 B1**
(45) Date of publication and mention of the grant of the patent: **17.03.2021**
(21) Application number: 17700725.9
(22) Date of filing: 11.01.2017
(51) Int. Cl.: H04N 13/128, H04N 13/344, H04N 13/371, H04N 13/00, A61B 6/02, A61B 3/11

(54) **DETECTION SYSTEM**
DETEKTIONSSYSTEM
SYSTÈME DE DÉTECTION

(30) Priority: 12.01.2016 GB 201600572
(43) Date of publication of application: 21.11.2018
(73) Proprietor: Sony Interactive Entertainment Inc., Tokyo 108-0075 (JP)
(72) Inventor: RAGHOEBARDAJAL, Sharwin Winesh, London W1F 7LP (GB); BENSON, Simon Mark, London W1F 7LP (GB)
(74) Representative: D Young & Co LLP
(86) International application number: PCT/GB2017/050056
(87) International publication number: WO 2017/122004

(56) References cited:
- WO-A1-2015/007784
- US-A1- 2013 050 833

## Description

### BACKGROUND

### Field of the Disclosure

This disclosure relates to detection systems.

### Description of the Prior Art

The "background" description provided herein is for the purpose of generally presenting the context of the disclosure. Work of the presently named inventors, to the extent it is described in this background section, as well as aspects of the description which may not otherwise qualify as prior art at the time of filing, are neither expressly or impliedly admitted as prior art against the present disclosure.

A head-mountable display (HMD) is one example of a head-mountable apparatus. In an HMD, an image or video display device is provided which may be worn on the head or as part of a helmet. Either one eye or both eyes are provided with small electronic display devices.

Although the original development of HMDs was perhaps driven by the military and professional applications of these devices, HMDs are becoming more popular for use by casual users in, for example, computer game or domestic computing applications.

HMDs can be used to view stereoscopic or other content. The successful portrayal of stereoscopic content to the user can depend, at least in part, on the extent to which display parameters of the content are matched to the eye separation (such as the inter-pupillary distance or IPD) of the HMD wearer. There is therefore a need for a system to detect the eye separation of a user.

The foregoing paragraphs have been provided by way of general introduction, and are not intended to limit the scope of the following claims. The described embodiments, together with further advantages, will be best understood by reference to the following detailed description taken in conjunction with the accompanying drawings.

Various aspects and features of the present disclosure are defined in the appended claims and within the text of the accompanying description and include at least a video server, a head mountable display, a system, a method of operating a video server or a head-mountable apparatus as well as a computer program and a video signal.

WO 2015/007784 A1 discloses using a depth image to determine the distance of the user's eyes with the help of a gauge.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:
Figure 1 schematically illustrates an HMD to be worn by a user;
Figure 2 is a schematic plan view of an HMD;
Figures 3 and 4 schematically illustrate a user wearing an HMD connected to a Sony® PlayStation ® games console;
Figure 5 schematically illustrates an arrangement for detecting a user's IPD;
Figure 6 is a schematic side view illustrating the arrangement of Figure 5, in use;
Figure 7 schematically illustrates a base computing device;
Figures 8a and 8b provide a schematic flowchart illustrating a detection process;
Figure 9 is a schematic flowchart illustrating a process for operating a head mountable display; and
Figure 10 schematically illustrates an example system.

### DESCRIPTION OF THE EMBODIMENTS

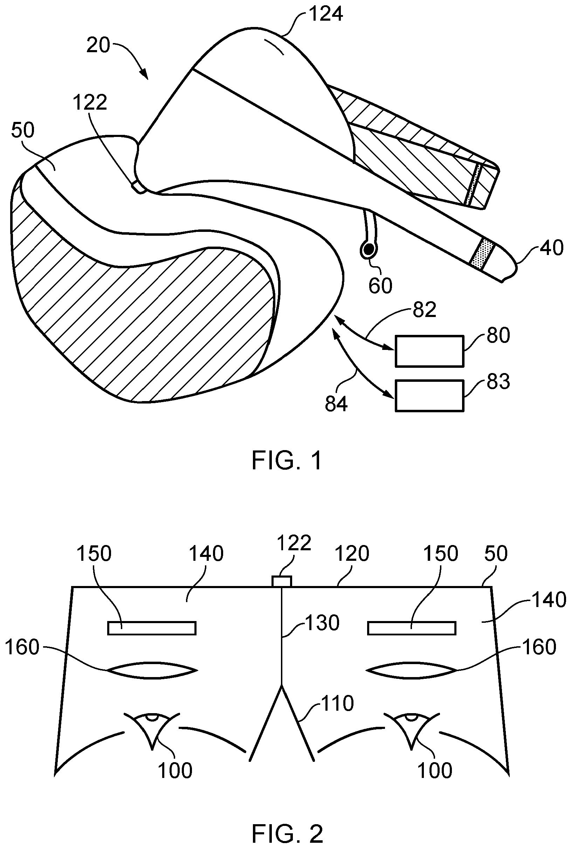

Referring now to Figure 1, an HMD 20 (as an example of a generic head-mountable apparatus) is wearable by a user. The HMD comprises a frame 40, in this example formed of a rear strap and an upper strap, and a display portion 50.

Note that the HMD of Figure 1 may comprise further features, to be described below in connection with other drawings, but which are not shown in Figure 1 for clarity of this initial explanation.

The HMD of Figure 1 completely (or at least substantially completely) obscures the user's view of the surrounding environment. All that the user can see is the pair of images displayed within the HMD, one image for each eye.

The HMD has associated headphone audio transducers or earpieces 60 which fit into the user's left and right ears. The earpieces 60 replay an audio signal provided from an external source, which may be the same as the video signal source which provides the video signal for display to the user's eyes.

The combination of the fact that the user can see only what is displayed by the HMD and, subject to the limitations of the noise blocking or active cancellation properties of the earpieces and associated electronics, can hear only what is provided via the earpieces, mean that this HMD may be considered as a so-called "full immersion" HMD. Note however that in some embodiments the HMD is not a full immersion HMD, and may provide at least some facility for the user to see and/or hear the user's surroundings. This could be by providing some degree of transparency or partial transparency in the display arrangements, and/or by projecting a view of the outside (captured using a camera, for example a camera mounted on the HMD) via the HMD's displays, and/or by allowing the transmission of ambient sound past the earpieces and/or by providing a microphone to generate an input sound signal (for transmission to the earpieces) dependent upon the ambient sound.

A front-facing camera 122 may capture images to the front of the HMD, in use. A Bluetooth® antenna 124 may provide communication facilities or may simply be arranged as a directional antenna to allow a detection of the direction of a nearby Bluetooth transmitter.

In operation, a video signal is provided for display by the HMD. This could be provided by an external video signal source 80 such as a video games machine or data processing apparatus (such as a personal computer), in which case the signals could be transmitted to the HMD by a wired or a wireless connection 82. Examples of suitable wireless connections include Bluetooth® connections. The external apparatus could communicate with a video server. Audio signals for the earpieces 60 can be carried by the same connection. Similarly, any control signals passed from the HMD to the video (audio) signal source may be carried by the same connection. Furthermore, a power supply 83 (including one or more batteries and/or being connectable to a mains power outlet) may be linked by a cable 84 to the HMD. Note that the power supply 83 and the video signal source 80 may be separate units or may be embodied as the same physical unit. There may be separate cables for power and video (and indeed for audio) signal supply, or these may be combined for carriage on a single cable (for example, using separate conductors, as in a USB cable, or in a similar way to a "power over Ethernet" arrangement in which data is carried as a balanced signal and power as direct current, over the same collection of physical wires). The video and/or audio signal may be carried by, for example, an optical fibre cable. In other embodiments, at least part of the functionality associated with generating image and/or audio signals for presentation to the user may be carried out by circuitry and/or processing forming part of the HMD itself. A power supply may be provided as part of the HMD itself.

Some embodiments of the disclosure are applicable to an HMD having at least one electrical and/or optical cable linking the HMD to another device, such as a power supply and/or a video (and/or audio) signal source. So, embodiments of the disclosure can include, for example:
(a) an HMD having its own power supply (as part of the HMD arrangement) but a cabled connection to a video and/or audio signal source;
(b) an HMD having a cabled connection to a power supply and to a video and/or audio signal source, embodied as a single physical cable or more than one physical cable;
(c) an HMD having its own video and/or audio signal source (as part of the HMD arrangement) and a cabled connection to a power supply;
(d) an HMD having a wireless connection to a video and/or audio signal source and a cabled connection to a power supply; or
(e) an HMD having no cabled connections, having its own power supply and either or both of: its own video and/or audio source and a wireless connection to another video and/or audio source.

If one or more cables are used, the physical position at which the cable 82 and/or 84 enters or joins the HMD is not particularly important from a technical point of view. Aesthetically, and to avoid the cable(s) brushing the user's face in operation, it would normally be the case that the cable(s) would enter or join the HMD at the side or back of the HMD (relative to the orientation of the user's head when worn in normal operation). Accordingly, the position of the cables 82, 84 relative to the HMD in Figure 1 should be treated merely as a schematic representation.

Accordingly, the arrangement of Figure 1 provides an example of a head-mountable display system comprising a frame to be mounted onto an observer's head, the frame defining one or two eye display positions which, in use, are positioned in front of a respective eye of the observer and a display element mounted with respect to each of the eye display positions, the display element providing a virtual image of a video display of a video signal from a video signal source to that eye of the observer.

Figure 1 shows just one example of an HMD. Other formats are possible: for example an HMD could use a frame more similar to that associated with conventional eyeglasses, namely a substantially horizontal leg extending back from the display portion to the top rear of the user's ear, possibly curling or diverting down behind the ear. In other (not full immersion) examples, the user's view of the external environment may not in fact be entirely obscured; the displayed images could be arranged so as to be superposed (from the user's point of view) over the external environment.

In the example of Figure 1, a separate respective display is provided for each of the user's eyes. A schematic plan view of how this is achieved is provided as Figure 2, which illustrates the positions 100 of the user's eyes and the relative position 110 of the user's nose. The display portion 50, in schematic form, comprises an exterior shield 120 to mask ambient light from the user's eyes and an internal shield 130 which prevents one eye from seeing the display intended for the other eye. The combination of the user's face, the exterior shield 120 and the interior shield 130 form two compartments 140, one for each eye. In each of the compartments there is provided a display element 150 and one or more optical elements 160. These can cooperate to display three dimensional or two dimensional content.

In some situations, an HMD may be used simply to view movies, or other video content or the like. If the video content is panoramic (which, for the purposes of this description, means that the video content extends beyond the displayable area of the HMD so that the viewer can, at any time, see only a portion but not all of the video content), or in other uses such as those associated with virtual reality (VR) or augmented reality (AR) systems, the user's viewpoint can be arranged to track movements with respect to a real or virtual space in which the user is located.

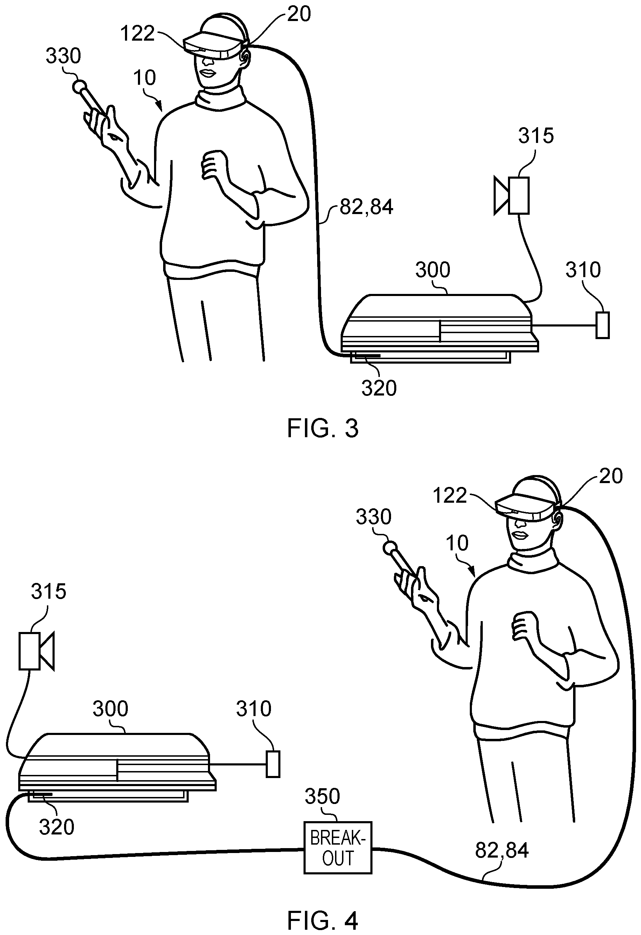

Figure 3 schematically illustrates a user wearing an HMD connected to a Sony® PlayStation® games console 300 as an example of a base device. The games console 300 is connected to a mains power supply 310 and (optionally) to a main display screen (not shown). A camera 315 such as a stereoscopic camera may be provided. A cable, acting as the cables 82, 84 discussed above (and so acting as both power supply and signal cables), links the HMD 20 to the games console 300 and is, for example, plugged into a USB socket 320 on the console 300. Note that in the present embodiments, a single physical cable is provided which fulfils the functions of the cables 82, 84. In Figure 3, the user is also shown holding a hand-held controller 330 which may be, for example, a Sony® Move® controller which communicates wirelessly with the games console 300 to control (or to contribute to the control of) operations relating to a currently executed program at the games console.

The video displays in the HMD 20 are arranged to display images provided via the games console 300, and the earpieces 60 in the HMD 20 are arranged to reproduce audio signals generated by the games console 300. The games console may be in communication with a video server. Note that if a USB type cable is used, these signals will be in digital form when they reach the HMD 20, such that the HMD 20 comprises a digital to analogue converter (DAC) to convert at least the audio signals back into an analogue form for reproduction.

Images from the camera 122 mounted on the HMD 20 are passed back to the games console 300 via the cable 82, 84. Similarly, if motion or other sensors are provided at the HMD 20, signals from those sensors may be at least partially processed at the HMD 20 and/or may be at least partially processed at the games console 300.

The USB connection from the games console 300 also provides power to the HMD 20, according to the USB standard.

Figure 4 schematically illustrates a similar arrangement in which the games console is connected (by a wired or wireless link) to a so-called "break out box" acting as a base or intermediate device 350, to which the HMD 20 is connected by a cabled link 82, 84. The breakout box has various functions in this regard. One function is to provide a location, near to the user, for some user controls relating to the operation of the HMD, such as (for example) one or more of a power control, a brightness control, an input source selector, a volume control and the like. Another function is to provide a local power supply for the HMD (if one is needed according to the embodiment being discussed). Another function is to provide a local cable anchoring point. In this last function, it is not envisaged that the break-out box 350 is fixed to the ground or to a piece of furniture, but rather than having a very long trailing cable from the games console 300, the break-out box provides a locally weighted point so that the cable 82, 84 linking the HMD 20 to the break-out box will tend to move around the position of the break-out box. This can improve user safety and comfort by avoiding the use of very long trailing cables.

It will be appreciated that the localisation of processing in the various techniques described in this application can be varied without changing the overall effect, given that an HMD may form part of a set or cohort of interconnected devices (that is to say, interconnected for the purposes of data or signal transfer, but not necessarily connected by a physical cable). So, processing which is described as taking place "at" one device, such as at the HMD, could be devolved to another device such as the games console (base device) or the break-out box. Processing tasks can be shared amongst devices. Source (for example, sensor) signals, on which the processing is to take place, could be distributed to another device, or the processing results from the processing of those source signals could be sent to another device, as required. So any references to processing taking place at a particular device should be understood in this context.

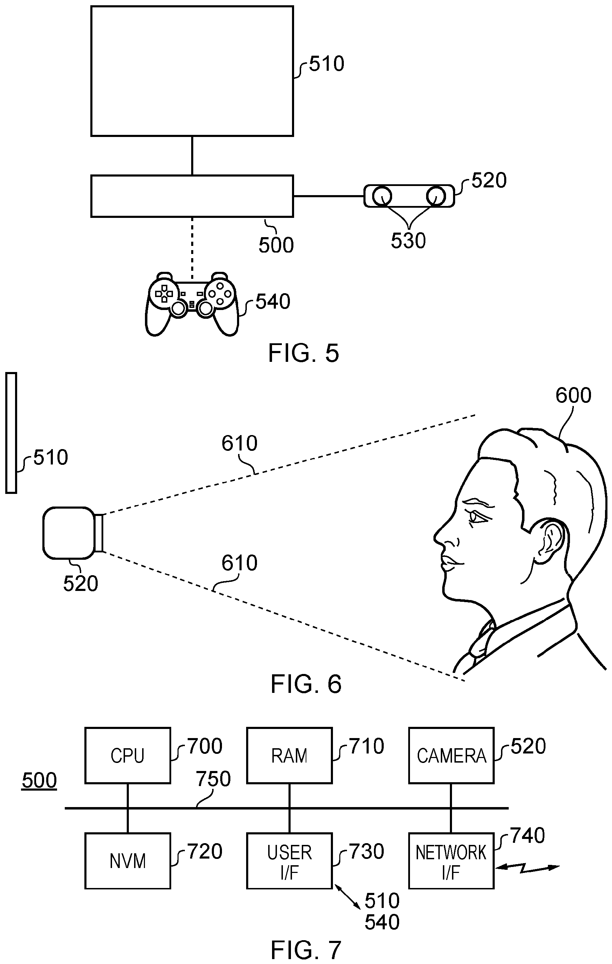

Figure 5 schematically illustrates an arrangement for detecting a user's inter-pupillary distance or IPD.

Detecting the IPD is an example of more generically detecting the user's eye separation, and is significant in the display of images by a head mountable display (HMD) system, particularly (though not exclusively) when three dimensional or stereoscopic images are being displayed.

As discussed above with reference to Figure 2, example HMDs use display elements which provide a separate image to each of the user's eyes. In instances where these separate images are left and right images of a stereoscopic image pair, the illusion of depth or three dimensions can be provided. However, if the lateral separation of the display positions of the left and right images is different to the user's IPD, this can result in the portrayed depths not appearing to be correct to the currently viewing user or in some instances a partial breakdown of the three dimensional illusion can be caused, potentially leading to user discomfort in the viewing process. In order to achieve a good three dimensional illusion when displaying images to a user with an HMD, the lateral separation of the two images should be reasonably well matched (for example, within (say) 1mm) to the user's IPD.

Therefore, an arrangement to detect the user's IPD can be a useful part of an HMD system, although of course it can stand on its own as an IPD detection arrangement.

In examples, given that the IPD of a particular user is extremely unlikely to change once it has been properly measured, a user can store his or her IPD details against a user account or similar identification, so that the measurement needs to be taken only once for each user, and then the measurement can be recalled for subsequent operation by that user.

In particular, Figure 5 schematically illustrates a base computing device 500, which may be a device such as the games console 300 of Figures 3 and 4 or may be another computing device, a display screen 510, a stereoscopic camera 520 connected to or otherwise associated with the base computing device so that the base computing device can receive and process images captured by the stereoscopic camera 520, the stereoscopic camera 520 including left and right image capture devices 530, and a user controller 540.

Note that the stereoscopic camera 520 is just an example of a depth camera which acquires depth data associated with a captured image. A stereoscopic camera does this by acquiring an image pair (for example a left/right image pair), for example at the same instant in time (though arrangements at which the images of the image pair are acquired at different temporal instants are envisaged). Other arrangements can make use of depth detection techniques such as the projection and acquisition of so-called structured light - a pattern of (for example) infra-red radiation which can be projected onto a scene, so that an acquired infra-red image of the scene can be used to derive depth information from the reflected pattern of structured light. In other arrangements other depth detection techniques such as acoustic sonar or radio frequency radar detection could be used. In cases where a single image and an associated set of depth information such as a depth map is acquired, left and right images can be derived from the image and the depth information. This type of technique is also referred to as acquiring a stereoscopic image or image pair.

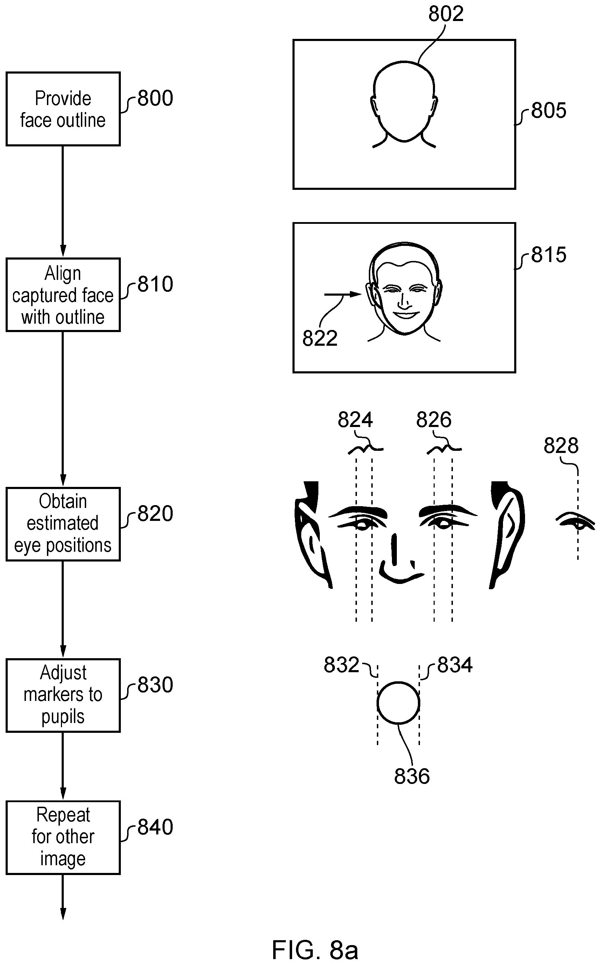

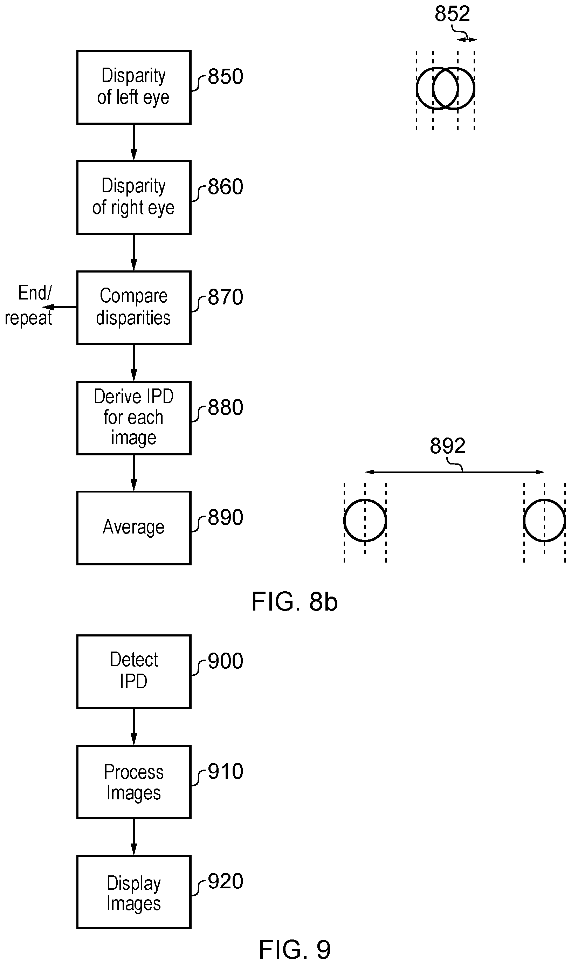

Figure 6 is a schematic side view illustrating the arrangement of Figure 5, in use. In Figure 6, the stereoscopic camera 520 captures an image pair of a current user 600 (in particular, of the user's face) according to a field of view illustrated schematically by lines 610. The display screen 510 is within view of the user 600 in readiness for operations to be described below with reference to Figures 8a and 8b.

Figure 7 schematically illustrates a base computing device such as the device 500 in more detail.

The base computing device comprises one or more central processing units (CPUs) 700; random access memory (RAM) 710; non-volatile memory (NVM) 720 such as read only memory (ROM), flash memory, hard disk storage or the like; a user interface 730 connectable, for example, to the display 510 and the controller 540; the camera 520 and a network interface 740 connectable, for example, to an internet connection. These components are linked by a bus arrangement 750. In operation, computer software, which may be provided via the network interface 740 or via the non-volatile memory 720 (for example, by a removable disk) is executed by the CPU 700 with data and program instructions being stored, as appropriate, by the RAM 710. It will be appreciated that the computer software may perform one or more steps of the methods to be discussed below. It will also be appreciated that such computer software, and/or a medium by which the computer software is provided (such as a non-volatile machine-readable storage medium such as a magnetic or optical disk) are considered to be embodiments of the present disclosure.

Figures 8a and 8b together provide a schematic flowchart illustrating a detection process. The end of the process described with reference to Figure 8a forms the start of the process described with reference to Figure 8b, so that the two drawings (Figures 8a and 8b) cooperate to provide a single composite flowchart.

The left hand portion of Figures 8a and 8b provides schematic flowchart steps, and the right hand side provides schematic images to illustrate the operation of corresponding flowchart steps.

Referring to Figure 8a, at a step 800 the device 500 generates an outline 802 of a face for display on the display screen 510. The outline 802 is superposed over the live feed image.

At a step 810, the user 600 moves with respect to the field of view of the camera 520 so as to align a captured image (for example, a stereoscopic image pair) of the user 600's face with the outline 802, both in terms of position within the captured image and size within the captured image.

Accordingly, the steps 800, 810 provide one example of a technique for obtaining a generally well-aligned and suitably sized image pair of the user's face by the camera 520. In other examples, a snapshot (single) or other image pair of the user's face can be captured and, for example, face detection techniques used to detect the position of the face image and to resize the image(s) if necessary or appropriate.

Once the captured face is appropriately aligned with the outline, a snapshot or single image pair can be captured of the face, either in response to a user command (when the user is satisfied that the alignment is correct) or in response to an automatic detection that the alignment is correct. This single captured image pair can be used as the basis of the remainder of the technique to be discussed below. In other examples, ongoing captured image pairs (a video feed) could be used as the basis of the subsequent steps.

At a step 820, the device 500 obtains estimated eye positions from one of the captured image pair. In an example, a left-to-right (or right-to-left) scan is carried out at a vertical image position 822 in one of the image pair corresponding to an expected eye position within the outline 802, scanning for aspects which are characteristic of a user's eyes. For example, such aspects could include a portion of skin-tone, followed by a portion of white or near-white (corresponding to the sclera or "whites" of the eyes) followed by a coloured portion corresponding to the iris, followed by a dark portion corresponding to the pupil and so on. If such aspects are not found in an appropriate order or configuration, then the device 500 can vary the image height 822 and repeat the test.

In other examples, face detection techniques may be used to model the face as captured by the captured image, with such techniques providing an approximation or estimate of where the eyes are located.

The result of the step 820 is, in one example, a pair of sets of boundaries 824, 826 indicating left and right boundaries of each eye's estimated position. In an alternative, a pupil centre (indicated by a respective pupil centre marker 828) could be detected for each eye as an eye feature.

At a step 830, the user is requested (for example, by a displayed indication on the display screen 510) to adjust the boundary markers 824, 826 or the pupil centre marker 828 to the left and right extent of the user's pupils in the captured image, for example using one or more controls on the controller 540. The user can indicate (for example, by pressing a particular button such as an X button) that the process has been completed to the user's satisfaction.

Note that in some examples both of the steps 820, 830 are carried out. In other examples, one or other (but not both) of these two steps can be carried out, which is to say the process could be automatic with manual refinement, or manual, or automatic to detect the eye positions within the captured image.

The result of the step 830 is, for each eye, a pair of boundary markers 832, 834 indicating the left and right extent of the user's pupil 836. Basing the process on the pupil (rather than the iris or the sclera) can provide a better estimation of the IPD at the end of the process. However, it will be appreciated that based on an assumption that the user is looking directly at the camera (or at another known or defined point, such as a point on the display screen 510) when the image is captured, the boundary markers 832, 834 could refer instead to the extent of the iris or the sclera.

The steps 820, 830 are carried out first for one of the stereoscopic image pair captured by the camera 520, and then at a step 840, the same two steps (820, 830) are repeated for the other of the two images. Note that the results obtained at the first image can be used to provide an assumption or initial approximation of the correct positioning in the other image.

It will be appreciated that in the case of a fully automated detection arrangement (the step 820 but not the step 830) there is no need to carry out the processing of the left and right images sequentially. Where a manual intervention (for step 830) is provided, it can be convenient to carry out the two steps (the detection of pupil positions in the left image and detection of pupil positions in the right image) sequentially, but again this is not strictly necessary and a split screen type of arrangement could be used to allow two versions (the left image and the right image) of the user's face to be displayed and handled simultaneously.

In examples, the process may be carried out so as to give four eye (for example, pupil centre) positions, once for each eye in each of the left and right images. Data obtained from one image may be used to approximate or steer the detection in the other of the image pair.

The process now continues with the flowchart of Figure 8b.

The steps described so far have resulted in the derivation of data indicating the pupil position for each of the user's left and right eyes, in each of the left and right images captured by the stereoscopic camera 520. Now, taking each eye in turn, the disparity (lateral difference in position) between the left image and the right image of that eye is detected at steps 850, 860. An example of the disparity 852 is also illustrated. Note, once again, that the steps 850, 860 can be carried out simultaneously or sequentially.

The disparities indicate the depth position in the captured stereoscopic (3D) image pair of each of the eyes. At a step 870, the disparities are compared, which is to say the disparity or depth for the left eye is compared with the disparity or depth for the right eye. Ideally, if the user had positioned the user's face perpendicular to the plane of the camera, the disparities are the same. If the disparities are very different, this could indicate that the user's eyes were not at the same distance from the camera, for example because the user held his or her head at an angle to the camera. If the compared disparities exceed a threshold difference between them, then at the step 870 the process is (i) terminated, or (ii) caused to repeat (which is to say, the user is requested to have another image captured), or (iii) compensated, which is to say that a compensation is applied so as to rotate the detected eye positions in 3D space to be equidistant from the camera.

If however the disparities are within a threshold difference, or if item (iii) was applied, then at a step 880 an IPD is derived for each of the left and right images based on the detected 3D positions of the respective eyes in that image. This could be derived on the assumption that the eyes are equidistant from the camera (which is to say, the test of the step 870 indicated that the disparities were within the threshold difference). Or it could be on the basis that item (iii), rotation of the detected eye positions, was applied. In either instance, a linear distance detected on the assumption that the eyes are equidistant from the camera can be used. In an alternative, the distance in 3D space between the detected eye positions can be used, which means that even if the eyes are not equidistant from the camera, the actual eye separation (rather than an incorrect planar projection of the eye separation) is detected. In this situation, it can still be useful to apply the test of step 870, but the threshold would be one at which the difference in disparity means that the skew or rotation of the eye positions is so great that the eye separation is not reliably detectable or the process introduces too great an error.

The eye separation (such as IPD) is obtained using this technique for each of the images of the image pair, which is to say the separation of left and right eyes in the left image is obtained, and the separation of left and right eyes in the right image is also obtained.

At a step 890, the detected IPDs for the left and right images are averaged to provide an output IPD 892.

The flowchart of Figures 8a and 8b therefore provides an example of a detection method comprising:
detecting (at the steps 820, 830, 840 for example) features (such as pupils, for example left and right peripheries of pupils or pupil centres) of a user's right eye and left eye in a stereoscopic image pair of the user;
detecting (at the steps 850, 860 for example) the image depths of the right eye and left eye features in the stereoscopic image pair;
comparing (at the step 870 for example) the detected depths for the right and left eye features; and
when the difference between the detected depths is less than a threshold difference, detecting (at the steps 880, 890 for example) the separation of the user's eyes from the separation of the three dimensional positions of the right eye and left eye features.

The step 830 provides an example of displaying an image indicating the detected positions of the one or more features; and providing a user control to adjust one or more of the detected position.

The steps 880, 890 provide an example of detecting a centre of each pupil from the detected left and right peripheries, in which the step of detecting the separation comprises detecting the separation of the detected pupil centres.

Note that in other examples, the detection and/or manual alignment could be directly relating to the pupil centres, in which case there is no need for a derivation at this stage in the process of deriving a pupil centre position from the peripheries.

The depth detection may take the form of the steps 850, 860 for example, involving detecting the image disparity of the features of the right eye between left and right images of the stereoscopic image; and detecting the image disparity of the features of the left eye between left and right images of the stereoscopic image.

The arrangement can operate with respect to already-captured images, but in examples the method comprises capturing the stereoscopic image (for example at the steps 800, 810, for example using the camera 520).

Figure 9 is a schematic flowchart illustrating a process for operating a head mountable display, comprising: at a step 900, detecting the user's IPD or eye separation, for example by the process of Figures 8a and 8b, at a step 910 processing images for display to the user according to the detected IPD, and at a step 920 displaying the processed images using a head mountable display such as the HMD 20.

The arrangement of Figures 5-7, for example when operated in accordance with the method of Figures 8a, 8b and/or 9, provides an example of detection apparatus comprising:
a feature detector to detect features of a user's right eye and left eye in a stereoscopic image pair of the user;
a depth detector to detect the image depths of the right eye and left eye features in the stereoscopic image pair;
a comparator to compare the detected depths for the right and left eye features; and
a separation detector to detect the separation of the user's eyes from the separation of the three dimensional positions of the right eye and left eye features, when the difference between the detected depths is less than a threshold difference.

As discussed, the apparatus may comprise the camera 520 or may operate with respect to already-captured stereoscopic images.

A head mountable display system (such as that shown in Figures 3 or 4, with the features of Figure 5) may comprise detection apparatus as defined above; and an image processor (as part of the HMD or base computing device) to process images for display by a head mountable display according to the detected separation of the user's eyes. The system may comprise the HMD itself.

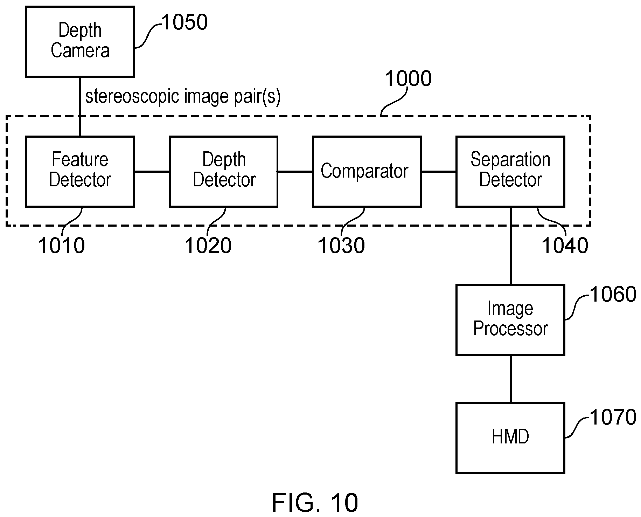

Figure 10 schematically illustrates an example system.

Detection apparatus 1000 comprises a feature detector 1010 to detect features of a user's right eye and left eye in a stereoscopic image pair of the user; a depth detector 1020 to detect the image depths of the right eye and left eye features in the stereoscopic image pair; a comparator 1030 to compare the detected depths for the right and left eye features; and a separation detector 1040 to detect the separation of the user's eyes from the separation of the three dimensional positions of the right eye and left eye features, when the difference between the detected depths is less than a threshold difference.

The detection apparatus may comprise a depth camera 1050 to acquire the stereoscopic image pair.

A head mountable display system may comprise the detection apparatus 1000 (optionally including the depth camera 1050); and an image processor 1060 to process images for display by a head mountable display according to the detected separation of the user's eyes.

The head mountable display system may also comprise a head mountable display 1070.

It will be apparent that numerous modifications and variations of the present disclosure are possible in light of the above teachings. It is therefore to be understood that within the scope of the appended claims, the disclosure may be practised otherwise than as specifically described herein.

## Claims

1. A detection method comprising:
detecting features of a user's right eye and left eye in a stereoscopic image pair of the user;
detecting image depths of the right eye and left eye features in the stereoscopic image pair;
comparing the detected depths for the right and left eye features; and
when a difference between the detected depths is less than a threshold difference, detecting a separation of the user's eyes from a separation of three dimensional positions of the right eye and left eye features.

2. A method according to claim 1, in which the step of detecting the features comprises:
detecting one or more features of the pupils of the user's right eye and left eye.

3. A method according to claim 2, comprising:
displaying an image indicating the detected positions of the one or more features; and
providing a user control to adjust one or more of the detected positions.

4. A method according to claim 2 or claim 3, in which the detected features are centres, or left and right peripheries, of each of the user's pupils.

5. A method according to claim 4, in which the step of detecting the separation of the user's eyes comprises:
detecting a centre of each pupil from the detected left and right peripheries;
and in which the step of detecting the separation of the user's eyes comprises detecting a separation of the detected pupil centres.

6. A method according to claim 1, in which the step of detecting the image depths comprises:
detecting image disparity of the features of the right eye between left and right images of the stereoscopic image pair; and
detecting image disparity of the features of the left eye between left and right images of the stereoscopic image pair.

7. A method according to any one of the preceding claims, comprising:
capturing the stereoscopic image pair.

8. A method according to any one of the preceding claims, comprising:
processing images for display by a head mountable display according to the detected separation of the user's eyes.

9. Computer software which, when executed by a computer, causes the computer to perform the method of any one of the preceding claims.

10. Detection apparatus (1000) comprising:
a feature detector (1010) to detect features of a user's right eye and left eye in a stereoscopic image pair of the user;
a depth detector (1020) to detect image depths of the right eye and left eye features in the stereoscopic image pair;
a comparator (1030) to compare the detected depths for the right and left eye features; and
a separation detector (1040) to detect a separation of the user's eyes from a separation of three dimensional positions of the right eye and left eye features, when a difference between the detected depths is less than a threshold difference.

11. Apparatus according to claim 10, comprising a depth camera (1050) to acquire the stereoscopic image pair.

12. A head mountable display system comprising:
detection apparatus (1000) according to claim 10 or claim 11; and
an image processor (1060) to process images for display by a head mountable display (1070) according to the detected separation of the user's eyes.

13. A head mountable display system according to claim 12, comprising:
a head mountable display (1070).

## Patentansprüche

1. Detektionsverfahren, umfassend:
Detektieren von Merkmalen eines rechten Auges und linken Auges eines Benutzers in einem stereoskopischen Bildpaar des Benutzers;
Detektieren von Bildtiefen von Merkmalen des rechten Auges und des linken Auges in dem stereoskopischen Bildpaar;
Vergleichen der detektierten Tiefen der Merkmale des rechten und des linken Auges; und
wenn eine Differenz zwischen den detektierten Tiefen unter einer Schwellenwertdifferenz liegt, Detektieren eines Abstands der Augen des Benutzers von einem Abstand von dreidimensionalen Positionen der Merkmale des rechten Auges und des linken Auges.

2. Verfahren nach Anspruch 1, wobei der Schritt des Detektierens der Merkmale umfasst: Detektieren von einem oder mehreren Merkmalen der Pupillen von dem rechten Auge und dem linken Auge des Benutzers.

3. Verfahren nach Anspruch 2, umfassend:
Anzeigen eines Bildes, welches die detektierten Positionen des einen oder der mehreren Merkmale angibt; und
Bereitstellen einer Benutzersteuerung, um eine oder mehrere der detektierten Positionen anzupassen.

4. Verfahren nach Anspruch 2 oder Anspruch 3, wobei die detektierten Merkmale Zentren oder linke und rechte Ränder von jeder der Pupillen des Benutzers sind.

5. Verfahren nach Anspruch 4, wobei der Schritt des Detektierens des Abstands der Augen des Benutzers umfasst:
Detektieren eines Zentrums jeder Pupille aus den detektierten linken und rechten Rändern; und
wobei der Schritt des Detektierens des Abstands der Augen des Benutzers Detektieren eines Abstands der detektierten Pupillenzentren umfasst.

6. Verfahren nach Anspruch 1, wobei der Schritt des Detektierens der Bildtiefen umfasst:
Detektieren von Bildungleichheit der Merkmale des rechten Auges zwischen linken und rechten Bildern des stereoskopischen Bildpaares; und
Detektieren von Bildungleichheit der Merkmale des linken Auges zwischen linken und rechten Bildern des stereoskopischen Bildpaares.

7. Verfahren nach einem der vorhergehenden Ansprüche, umfassend:
Erfassen des stereoskopischen Bildpaares.

8. Verfahren nach einem der vorhergehenden Ansprüche, umfassend:
Verarbeiten von Bildern zur Anzeige durch eine kopfbefestigbare Anzeige gemäß dem detektierten Abstand der Augen des Benutzers.

9. Computersoftware, die bei Ausführung durch einen Computer herbeiführt, dass der Computer das Verfahren nach einem der vorhergehenden Ansprüche durchführt.

10. Detektionsgerät (1000), umfassend:
einen Merkmaldetektor (1010) zum Detektieren von Merkmalen eines rechten Auges und linken Auges eines Benutzers in einem stereoskopischen Bildpaar des Benutzers;
einen Tiefendetektor (1020) zum Detektieren von Bildtiefen von Merkmalen des rechten Auges und des linken Auges in dem stereoskopischen Bildpaar;
einen Komparator (1030) zum Vergleichen der detektierten Tiefen der Merkmale des rechten und des linken Auges; und
einen Abstandsdetektor (1040) zum Detektieren eines Abstands der Augen eines Benutzers aus einem Abstand von dreidimensionalen Positionen von Merkmalen des rechten Auges und des linken Auges, wenn eine Differenz zwischen den detektierten Tiefen kleiner als eine Schwellenwertdifferenz ist.

11. Gerät nach Anspruch 10, umfassend eine Tiefenkamera (1050) zum Erfassen des stereoskopischen Bildpaares.

12. Kopfbefestigbares Anzeigesystem, umfassend:
ein Detektionsgerät (1000) nach Anspruch 10 oder 11; und
einen Bildprozessor (1060) zum Verarbeiten von Bildern zur Anzeige durch eine kopfbefestigbare Anzeige (1070) gemäß dem detektierten Abstand der Augen eines Benutzers.

13. Kopfbefestigbares Anzeigesystem nach Anspruch 12, umfassend:
eine kopfbefestigbare Anzeige (1070).

## Revendications

1. Procédé de détection comprenant :
la détection de caractéristiques de l'œil droit et de l'œil gauche d'un utilisateur dans un couple d'images stéréoscopiques de l'utilisateur ;
la détection de profondeurs d'image des caractéristiques de l'œil droit et de l'œil gauche dans le couple d'images stéréoscopiques ;
la comparaison des profondeurs détectées pour les caractéristiques de l'œil droit et de l'œil gauche ; et
lorsqu'une différence entre les profondeurs détectées est inférieure à une différence seuil, la détection d'une séparation des yeux de l'utilisateur par rapport à une séparation des positions tridimensionnelles des caractéristiques de l'œil droit et de l'œil gauche.

2. Procédé selon la revendication 1, l'étape de détection des caractéristiques comprenant :
la détection d'une ou de plusieurs caractéristiques des pupilles de l'œil droit et de l'œil gauche de l'utilisateur.

3. Procédé selon la revendication 2, comprenant :
l'affichage d'une image indiquant les positions détectées de la ou des caractéristiques ; et
la fourniture d'une commande de l'utilisateur pour ajuster une ou plusieurs des positions détectées.

4. Procédé selon la revendication 2 ou la revendication 3, les caractéristiques détectées étant les centres ou les périphéries gauche et droite de chacune des pupilles de l'utilisateur.

5. Procédé selon la revendication 4, l'étape de détection de la séparation des yeux de l'utilisateur comprenant :
la détection d'un centre de chaque pupille à partir des périphéries gauche et droite détectées ;
et l'étape de détection de la séparation des yeux de l'utilisateur comprenant la détection d'une séparation des centres des pupilles détectées.

6. Procédé selon la revendication 1, l'étape de détection des profondeurs d'image comprenant :
la détection de la disparité des caractéristiques de l'œil droit entre les images gauche et droite du couple d'images stéréoscopiques ; et
la détection de la disparité des caractéristiques de l'œil gauche entre les images gauche et droite du couple d'images stéréoscopiques.

7. Procédé selon l'une quelconque des revendications précédentes, comprenant :
la capture du couple d'images stéréoscopiques.

8. Procédé selon l'une quelconque des revendications précédentes, comprenant :
le traitement des images pour leur affichage par un dispositif d'affichage montable sur la tête en fonction de la séparation détectée des yeux de l'utilisateur.

9. Logiciel informatique qui, lorsqu'il est exécuté par un ordinateur, amène l'ordinateur à exécuter le procédé selon l'une quelconque des revendications précédentes.

10. Appareil de détection (1000) comprenant :
un détecteur de caractéristiques (1010) pour détecter des caractéristiques de l'œil droit et de l'œil gauche d'un utilisateur dans une paire d'images stéréoscopiques de l'utilisateur ;
un détecteur de profondeur (1020) pour détecter des profondeurs d'image des caractéristiques de l'œil droit et de l'œil gauche dans la paire d'images stéréoscopiques ;
un comparateur (1030) pour comparer les profondeurs détectées pour les caractéristiques de l'œil droit et de l'œil gauche ; et
un détecteur de séparation (1040) pour détecter une séparation des yeux de l'utilisateur par rapport à une séparation des positions tridimensionnelles des caractéristiques de l'œil droit et de l'œil gauche, lorsqu'une différence entre les profondeurs détectées est inférieure à une différence seuil.

11. Appareil selon la revendication 10, comprenant une caméra de profondeur (1050) pour acquérir le couple d'images stéréoscopiques.

12. Système d'affichage montable sur la tête comprenant :
un appareil de détection (1000) selon la revendication 10 ou la revendication 11 ; et
un processeur d'images (1060) pour traiter les images en vue de leur affichage par un dispositif d'affichage montable sur la tête (1070) en fonction de la séparation détectée des yeux de l'utilisateur.

13. Système d'affichage montable sur la tête selon la revendication 12, comprenant :
un dispositif d'affichage montable sur la tête (1070).
